Europäisches Patentamt

(19) European Patent Office

Office européen des brevets

(11) Publication number: **0 153 036**
**B1**

(12) **EUROPEAN PATENT SPECIFICATION**

(45) Date of publication of patent specification: **19.11.87**

(51) Int. Cl.⁴: **C 07 C 139/00, C 07 C 143/12**

(21) Application number: **85300555.1**

(22) Date of filing: **28.01.85**

(54) Sulphonation of fatty acid esters.

(30) Priority: **30.01.84 GB 8402363**

(43) Date of publication of application:
**28.08.85 Bulletin 85/35**

(45) Publication of the grant of the patent:
**19.11.87 Bulletin 87/47**

(84) Designated Contracting States:
**AT BE CH DE FR GB IT LI NL SE**

(56) References cited:
**EP-A-0 071 808**
**DE-A-3 309 049**
**DE-B-1 418 887**
**US-A-3 997 576**

(73) Proprietor: **UNILEVER PLC**
**Unilever House Blackfriars P.O. Box 68**
**London EC4P 4BQ (GB)**
(84) **GB**

(73) Proprietor: **UNILEVER NV**
**Burgemeester s'Jacobplein 1 P.O. Box 760**
**NL-3000 DK Rotterdam (NL)**
(84) **BE CH DE FR IT LI**

(72) Inventor: **Roberts, David William**
**26 Venables Drive**
**Bebington Wirral Merseyside L63 9LT (GB)**

(74) Representative: **Roscoe, Brian Corrie et al**
**UNILEVER PLC Patents Division P.O. Box 68**
**Unilever House**
**London EC4P 4BQ (GB)**

EP 0 153 036 B1

Courier Press, Leamington Spa, England.

# 0 153 036

**Description**

Background to the invention

This invention relates to the sulphonation of fatty acid esters to produce materials of use as detergent actives. These actives are of value in detergent products without restriction to the product form. That is they are of use in detergent powders, liquids, pastes and bars. These detergent actives are usable in association with other detergent active classes and other additives and components of detergent formulations.

Summary of invention

The invention is directed to a method of manufacturing fatty acid ester sulphonates utilising a sulphur trioxide/inert gas sulphonating agent. The method of the present invention allows the production of fatty acid ester sulphonates having a relatively low content of fatty acid sulphonates. The latter are also detergent active species but the detergent manufacturer desiring to use fatty acid ester sulphonates for their specific properties would require to have the level of fatty acid sulphonates as low as possible because the properties of the latter actives are not compatible with those of the fatty acid ester sulphonate material.

The general method of the invention is the manufacture of fatty acid (C8—C22) ester (C1—C4) sulphonates wherein:

i) fatty acid esters are sulphonated with a sulphur trioxide/inert gas mixture in a molar ratio in the range from about 1 to 1 to about 2.5 to 1 $SO_3$/ester in the temperature range 0°C—150°C, preferably 30°C to 130°C.

ii) the initial reaction mixture so produced is subjected to an ageing step during which the sulphonation of the fatty acid ester to alpha sulphonated material proceeds substantially to completion and

iii) the resulting sulphonic acid is subsequently neutralised.

The present invention proposes addition of short chain (C1—C4) alcohol to the sulphonic acid mixture, in the absence of a bleaching species, in an amount of at least about 25 mole % of the sulphur trioxide used in excess of 1:1 $SO_3$:ester stoichiometry at a point not before the end of the ageing step. The addition of the short chain alcohol at this point reduces the content of fatty acid sulphonate in the resulting product.

The added alcohol reacts rapidly and no specific ageing period is required for completion of the reaction. However, in practice a period usually elapses before neutralisation. The most preferred amount of alcohol added is 100 mole % of the excess sulphur trioxide but a substantial benefit is also obtained when the amount is at least about 50 mole %. No additional benefit is obtained by adding more than 100 mole % alcohol but there is no bar against such addition although the process economics could be affected.

The short chain alcohol must not be added to the sulphonated product before sulphonation of fatty acid ester to alpha sulphonated material is substantially complete; this point can be identified by monitoring the reaction medium by NMR to detect unsulphonated species.

No additional benefit is obtained by continuing to heat the reaction mixture beyond this point before adding alcohol, but there is no bar to such further heating. The period of the ageing step will reduce with the use of higher molar levels of the sulphur trioxide in the sulphonating mixture and with higher ageing temperature. For example when step (i) is performed at 60°C with a mole ratio of 2:1 for $SO_3$:ester the ageing period is almost zero, whereas for sulphonation of methyl laurate at 70°C with a 1.3 to 1 mole ratio of $SO_3$/ester it is about 30—45 minutes.

The addition of short chain alcohol to the sulphonic acid mix is performed in the absence of a bleaching species. In practice if the sulphonic acid was bleached the appropriate bleaching species used at this stage would be aqueous hydrogen peroxide. The necessary presence of water with the use of this bleaching species would dilute and complete with the short chain alcohol and make it less effective. Thus there is a process benefit in separating the short chain alcohol addition from the bleaching stage. Bleaching can be performed immediately before the neutralisation stage or on the neutralised product e.g. with hypochlorite.

Prior literature

Smith & Stirton (JAOCS 44-1967-405) describe the sulphonation of fatty acid esters using liquid sulphur trioxide at low temperatures. Some samples of the sulphonation product were refluxed for prolonged periods with a relatively large quantity of an appropriate alcohol to provide re-esterification ⸳ before re-crystallisation.

US 4404143 (Lion Corporation) describes the preparation of fatty acid ester sulphonates in which short chain alcohols are present in the bleaching and neutralisation stages.

US 3997576 (Lion Fat and Oil) describes sulphonation of a mixture of fatty acid ester and short chain alcohol.

Components

The feedstock fatty acid (C8—C22) esters (C1—C4) will usually be derived from naturally occurring fatty acids but petroleum derived, i.e. synthetic, fatty acids can also be used. Examples of the sources of glycerides from which suitable fatty acids can be obtained are coconut oil, tallow, palm oil, palm kernel oil, fish oils, soya and babassu. The fatty acids will usually be hardened to an Iodine Value of 1 or lower to

2

reduce the formation of coloured species during sulphonation. Some oils, e.g. tallow, soya, and some fish oils, will require hydrogenation to provide suitable fatty acids.

The short chain alcohols forming the ester group will usually be methanol and ethanol, but other short chain alcohols, e.g. n-propanol, iso-propanol, n-butanol and sec-butanol can be used.

The sulphonation is performed using a sulphur trioxide/inert gas mixture. The diluent gas, which is normally air, is required to be inert in the sulphonation reaction; nitrogen is another suitable diluent gas. There is no criticality in the source of the sulphur trioxide.

Examples

Examples of the process of the invention will now be given by way of illustration.

Laboratory scale ester sulphonation experiments were designed to simulate a batch process in a stirred tank reactor. The reaction vessel was a Pyrex 3-necked flask fitted with a stirrer, a reflux condenser and a gas inlet tube for introduction of $SO_3$/air below the surface of the liquid being sulphonated. Gaseous $SO_3$ was produced by adding liquid $SO_3$ from a burette to a vaporiser, consisting of a Pyrex flask heated at 100° and connected to a supply of dry nitrogen and to the reaction vessel. $SO_3$:ester mole ratios were calculated from the amount of liquid $SO_3$ fed to the vapouriser and the weight of ester used.

After addition of $SO_3$, was complete (ca. 60 min.) the acid mix was aged (Stage I) for a determined period at a temperature approximately equal to the sulphonation temperature, then alcohol was added with stirring and the reaction mixture maintained at a defined temperature (Stage II). A bleaching species was not present during the reaction of the short chain alcohol with the acid mix. The reaction product was then neutralised by pouring it, simultaneously with aqueous 2N sodium hydroxide, into water, with vigorous stirring and monitoring of pH by means of a pH electrode, to give a solution at pH 6.5—7.5 of 10—40% detergent active.

TABLE I

| Example | Ester | SO$_3$:ester mole ratio | Stage I ageing | Stage II | Conversion (%) to detergent active | FAES/SFAS mole ratio in neutralised product |
|---------|-------|------------------------|----------------|----------|-----------------------------------|---------------------------------------------|
| I | Methyl laurate | 1.3:1 | 80°/45 min | MeOH, 0.3:1/45 min | 100 | 96/4 |
| II | Methyl laurate | 1.3:1 | 70°/42 min | MeOH, 0.5:1/10 min | 98.5 | 99/1 |
| III | Ethyl laurate | 1.3:1 | 80°/45 min | EtOH, 1:1/45 min | 95 | >90% Ethyl FAES |
| IV | Methyl stearate | 1.55:1 | 90°/35 min | MeOH, 0.4:1/5 min | 100 | >95% FAES |
| V | Methyl laurate | 1.3:1 | 70°/30 min | EtOH, 0.3:1/5 min | 96 | 91*/9 |
| VI (control) | Methyl laurate | 1.3:1 | 70°/30 min | 0.3:1/5 min | 96 | 70/30 |

*Of which 79 parts methyl sulpholaurate and 12 parts ethyl sulpholaurate

Quantitative analysis on the neutralised reaction products was carried out by hydrogen NMR, using a Bruker WM 360 instrument operating at 360 Mhz.

Table I quotes the results obtained with five examples (I to V) of this procedure. The Stage I ageing is described by the temperature (°C) and period (minutes) used and Stage II by the alcohol added, the mole ratio of alcohol to initial ester content and period (minutes) used. The mole ratio of fatty acid ester sulphonate (FAES) to fatty acid sulphonate (SFAS) in the neutralised product is given in the final column.

Example VI is a control procedure following the process features of Example V but omitting the alcohol addition.

**Claims**

1. A method of manufacturing fatty acid (C8—C22) ester (C1—C4) sulphonates wherein:

i) fatty acid esters are sulphonated with a sulphur trioxide/inert gas mixture in a molar ratio in the range from 1 to 1 to 2.5 to 1 $SO_3$/ester in the temperature range 0°C—150°C,

ii) the initial reaction mixture so produced is subjected to an ageing step during which the sulphonation of the fatty acid ester to alpha-sulphonated material proceeds substantially to completion and

iii) the resulting sulphonic acid is subsequently neutralised, characterised in that short chain (C1—C4) alcohol is added to the sulphonic acid mixture, in the absence of a bleaching species, in an amount of at least 25 mole % of the sulphur trioxide used in excess of 1:1 $SO_3$:ester stoichiometry at a point not before the end of the ageing step.

2. A method according to claim 1 wherein the temperature of sulphonation is above 30°C.

3. A method according to claim 1 or 2 wherein the temperature of sulphonation is not above 130°C.

4. A method according to any preceding claim wherein the short chain alcohol is added in an amount of at least about 50 mole % of the sulphur trioxide excess.

5. A method according to claim 4 wherein the amount of short chain alcohol added approximates the sulphur trioxide excess.

**Patentansprüche**

1. Verfahren zur Herstellung von Fettsäure (C8—C22) ester (C1—C4) sulfonaten, worin:

(i) Fettsäureester mit einer Mischung aus Schwefeltrioxid/inertem Gas in einem molaren Verhältnis im Bereich von 1:1 bis 2,5:1 $SO_3$/Ester im Temperaturbereich von 0°C bis 150°C, sulfoniert werden,

(ii) die anfängliche, so hergestellte Reaktionsmischung einer Alterungsstufe unterworfen wird, während welcher die Sulfonierung des Fettsäureesters zu alpha-sulfoniertem Material im wesentlichen bis zur Vollendung fortschreitet und

(iii) die entstehende Sulfonsäure anschließend neutralisiert wird, dadurch gekennzeichnet, daß ein kurzkettiger (C1—C4) Alkohol zu der Sulfonsäuremischung zugesetzt wird, in Abwesenheit eines Bleichmittels, in einer Menge von mindestens 25 Mol-% des Schwefeltrioxids verwendet in Überschuß des stoichiometrischen Verhältnisses von 1:1 von $SO_3$:Ester zu einem Zeitpunkt nicht früher als dem Ende der Alterungsstufe.

2. Verfahren gemäß Anspruch 1, worin die Sulfonierungstemperatur oberhalb 30°C liegt.

3. Verfahren gemäß Anspruch 1 oder 2, worin die Sulfonierungstemperatur nicht oberhalb 130°C liegt.

4. Verfahren gemäß einem der vorgehenden Ansprüche, worin der kurzkettige Alkohol in einer Menge von mindestens etwa 50 Mol-% des Schwefeltrioxid-Überschusses zugesetzt wird.

5. Verfahren gemäß Anspruch 4, worin die Menge des zugesetzten kurzkettigen Alkohols etwa dem Schwefeltrioxid-Überschuß entspricht.

**Revendications**

1. Procédé de fabrication d'esters ($C_{1-4}$) d'acides gras ($C_{8-22}$) sulfonés, dans lequel:

(i) on sulfone les esters d'acides gras avec un mélange d'anhydride sulfurique et de gaz inerte dans un rapport molaire compris entre 1:1 et 2,5:1 $SO_3$/ester à une température comprise entre 0 et 150°C,

(ii) on soumet le mélange initial de réaction ainsi obtenu à un stade de vieillissement au cours duquel la sulfonation de l'ester d'acides gras en un produit alpha-sulfoné se poursuit sensiblement jusqu'à achèvement et

(iii) on neutralise ensuite l'acide sulfonique résultant, caractérisé en ce qu'on ajoute un alcool à chaîne courte ($C_{1-4}$) au mélange d'acide sulfonique en l'absence d'un composant de blanchiment à raison d'au moins 25 moles% par rapport à l'anhydride sulfurique en excès, de stoechiométrie $SO_3$:ester de 1:1, à un moment avant l'achèvement du stade de vieillissement.

2. Procédé selon la revendication 1, dans lequel la température de sulfonation est supérieure à 30°C.

3. Procédé selon la revendication 1 ou 2 dans lequel la température de sulfonation n'est pas supérieure à 130°C.

4. Procédé selon l'une quelconque des revendications précédentes, dans lequel on ajoute l'alcool à chaîne courte à raison d'au moins 50 moles% environ de l'excès d'anhydride sulfurique.

5. Procédé selon la revendication 4, dans lequel la quantité d'alcool à chaîne courte qu'on ajoute est voisine de l'excès d'anhydride sulfurique.

5